Europäisches Patentamt

European Patent Office

Office européen des brevets

(19)

(11) **EP 1 481 684 A1**

(12) **EUROPEAN PATENT APPLICATION**
published in accordance with Art. 158(3) EPC

(43) Date of publication:
**01.12.2004 Bulletin 2004/49**

(51) Int Cl.⁷: **A61K 38/16**, A61P 1/16,
A61P 3/06, A61P 3/10

(21) Application number: **03743531.0**

(22) Date of filing: **28.02.2003**

(86) International application number:
**PCT/JP2003/002366**

(87) International publication number:
**WO 2003/074071 (12.09.2003 Gazette 2003/37)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **01.03.2002 JP 2002055812
25.09.2002 JP 2002278880
25.09.2002 JP 2002278881**

(71) Applicant: **Nisshin Pharma Inc.
Tokyo 101-8441 (JP)**

(72) Inventors:
• **HORIGUCHI, Noboru c/o Horiguchi Clinic
Sakaide, Kagawa 762-0025 (JP)**
• **HORIGUCHI, Hiroshi c/o Horiguchi Clinic
Sakaide, Kagawa 762-0025 (JP)**
• **SUZUKI, Yoshio c/o Nisshin Pharma Inc.
Chiyoda-ku, Tokyo 101-8441 (JP)**

(74) Representative:
**Gille Hrabal Struck Neidlein Prop Roos
Patentanwälte,
Brucknerstrasse 20
40593 Düsseldorf (DE)**

(54) **REMEDIES FOR LIVER DISEASES, HYPERLIPEMIA AND DIABETES**

(57)    Drugs and foods for preventing and/or treating liver diseases, hyperlipemia and/or diabetes containing a peptide which is composed of L-amino acids constituting natural proteins, has an L-glutamine content of from 15 to 60% by mass and has an average molecular weight of from 200 to 100,000; and foods containing this peptide. By taking the above drugs or foods, effects of preventing or treating liver diseases, hyperlipemia and/or diabetes can be exhibited. These drugs and foods are highly efficacious and can be easily taken without any fear of side effects.

**Description**

Technical Field

The increasing number of patients with liver diseases, hyperlipemia or diabetes is becoming a serious social problem. Unique causative factors are known for certain forms of liver diseases, hyperlipemia and diabetes. For example, there are known infectious liver diseases such as viral hepatitis, primary hyperlipemia associated with a genetic defect, and diabetes caused by a genetic lack of insulin secretion. However, fatty liver, alcoholic hepatitis and other liver diseases, alimentary hyperlipemia and alimentary diabetes are usually also closely associated with overconsumption of starches, fats and alcoholic beverages as well as with living habits, and therefore the conditions of liver diseases, hyperlipemia and diabetes often occur together.

The present invention relates to prophylactic and/or therapeutic agents for liver diseases, hyperlipemia and/ or diabetes, and to prophylactic and/or therapeutic foods for liver diseases, hyperlipemia and/or diabetes. More specifically, the invention relates to prophylactic and/or therapeutic agents for liver diseases, hyperlipemia and/or diabetes characterized by comprising L-glutamine rich peptides, and to foods containing the peptides.

Background Art

Typical liver diseases include fatty liver, and inflammatory hepatitis such as viral hepatitis, alcoholic hepatitis and hepatic cirrhosis. Liver diseases are treated using glycyrrhizin formulations, polyenphosphatidylcholine, malotilate, diisopropylamine dichloroacetate, tiopronin, protoporphyrin sodium, methylmethioninesulfonium chloride, interferon, ursodesoxycholic acid, Shosai-koto, liver hydrolysate, glutathione or the like. However, these drugs have minimal ameliorating effects on liver diseases with short-term use, and must therefore be administered for longterm therapy. In addition, even in cases where some degree of rapid amelioration is achieved for liver diseases, the improved GOT, GPT and γ-GTP values are often no lower than the maximum values for the normal range. Moreover, even where therapeutic effects are exhibited on liver diseases, there have been problems of side effects.

It has therefore been desirable to develop highly safe drug agents and foods for prevention or treatment of liver diseases which are easily administerable on a daily basis and exhibit excellent therapeutic effects.

Hyperlipemia is the condition where total cholesterol or triglycerides, or both, exceed established standard values, and it includes primary hyperlipemia caused by a genetic defect, and secondary hyperlipemia resulting from alimentary causes, abnormal metabolism of sugars, proteins and fats, and primary liver diseases. Hyperlipemia is generally diagnosed if the total cholesterol level is 220 mg/dl or greater or the triglyceride level is 150 mg/dl or greater.

The form of hyperlipemia with the most problematic cause is secondary hyperlipemia resulting from excessive consumption of energy by intake of fat-rich foods such as meat, which contain high amounts of cholesterol or triglycerides, carbohydrate-rich foods and alcohol. Hyperlipemia is a cause of arteriosclerosis, which cannot be ignored because it leads to such serious conditions as myocardial infarction and cerebral thrombosis. Drug therapies are therefore administered to lower blood cholesterol or triglyceride levels, using such agents containing pravastatin sodium, cholestimide, simvastatin, nicotinic acid, clofibrate or the like. However, these drugs have been associated with drawbacks including side effects, discomfort during use and relapse after completion of administration.

Furthermore while diet and exercise regimens are also prescribed for treatment of hyperlipemia, such treatment methods must be followed for extensive periods before an effect is seen, and must therefore be continued at length. Consequently, it has been a desired goal to develop highly safe drug agents, as well as foods, for prevention and treatment of hyperlipemia which are easily administerable on a daily basis and exhibit excellent effects of lowering cholesterol or triglycerides. Certain polyphenols found in tea, red wine, cacao beans, etc., which are suitable for addition to foods, have been reported to have prophylactic effects against hypercholesterolemia (for example, see JP-11-308978-A1 and Journal of Japanese Society of Nutrition and Food Science 39(6), pp.495-500, 1986).

Diabetes is a syndrome characterized by chronic hyperglycemia due to insufficient insulin secretion or to insulin resistance, and it may be due to either genetic or environmental factors. Forms of diabetes include type I (insulin-dependent) diabetes wherein the pancreatic β cells are either destroyed or become dysfunctional for some reason and can no longer secrete insulin, thus resulting in hyperglycemia, type II (non-insulin-dependent) diabetes characterized by reduced insulin secretion, reduced insulin sensitivity in the tissues, etc., and pregnancy diabetes wherein the hormones secreted with pregnancy lower the action of insulin. Approximately 90% of diabetes cases are non-insulin-dependent and are considered to be due to genetic factors as well as other factors such as excessive energy intake, unbalanced diet, lack of exercise, stress and the like.

In any case, a condition of chronic hyperglycemia not only exacerbates the diabetes itself, but also produces complications such as neuropathy, retinopathy, kidney damage and the like, and can lead to serious diseases such as cerebrovascular disorders, ischemic heart disease, diabetic nephropathy, diabetic retinopathy and diabetic neuropathy. Pregnancy diabetes is also a cause of intrauterine fetal death or neonatal death.

Control of blood sugar levels is important for prevention or treatment of diabetes, and nutritional guidance, exercise therapy and drug treatments are employed for this purpose. Nutritional guidance and exercise therapy are effective means for preventing or treating diabetes, but because the effect is shown after long time, it is necessary to continuously follow a strict dietary restriction program or exercise regimen for an extended period of time. In addition, exercise therapy can not only provoke a hypoglycemic condition but can sometimes aggravate complications, depending on the patient, and must therefore be carried out with care.

Drug treatments are largely classified as either insulin-based therapies or oral diabetes remedies. Insulin-based therapies are mainly employed for patients with insulin-dependent diabetes, in which case the insulin must be injected on a daily basis with carefully controlled insulin injection doses. Oral diabetes remedies include insulin resistance ameliorants such as sulfonylurea-based remedies (SU agents), biguanide-based remedies (BG agents), $\alpha$-glycosidase inhibitors, pioglitazone hydrochloride and the like, but these'are associated with side effects such as hypoglycemia, lactic acidosis (nausea, vomiting, abdominal pain, coma) and hepatopathy, while care must also be taken with regard to concomitantly administered drugs. It has therefore been desirable to develop highly safe drug agents, as well as foods, for prevention and treatment of diabetes which are easily administerable on a daily basis and exhibit excellent effects of lowering blood sugar levels.

On the other hand, methods for obtaining L-glutamine or peptides containing it from wheat protein are known (for example, see JP-2019439-B1 and JP-2985193-B1). It has been reported that L-glutamine or peptides containing it are useful for maintaining the structure and function of the intestinal mucosa and for improving impairment of immunological function (for example, see JP-5-236909-A1 and EP756827-A2). However, it has not been hitherto known that L-glutamine rich peptides restore normal GOT, GPT and $\gamma$-GTP values, lower blood cholesterol and triglyceride levels, lower blood glucose levels, and have functions for prevention and/or treatment of liver diseases, hyperlipemia and/or diabetes.

Disclosure of the Invention

An object of the present invention is to provide drug agents for prevention and/or treatment of liver diseases, hyperlipemia and/or diabetes, as well as foods with effects of prevention and/or treatment of liver diseases, hyperlipemia and/or diabetes, which are highly efficacious, have low risk of side effects, are easily administerable and can be administered for long periods from the standpoint of both cost and safety.

The present inventors have discovered that administration of L-glutamine rich peptides improves the GOT, GPT and $\gamma$-GTP values of liver disease patients to normal or near-normal values within a short period of time, that administration of the peptides improves total cholesterol, LDL cholesterol, triglyceride and arteriosclerosis index values to normal or near-normal values within a short period of time, and that administration of the peptides improves blood glucose levels to normal or near-normal values within a short period of time.

The present invention therefore relates to prophylactic and/or therapeutic agents for liver diseases, hyperlipemia and/or diabetes characterized by comprising peptides composed of L-amino acids found in natural proteins, having an L-glutamine content of 15-60 wt% and an average molecular weight of 200-100,000. The peptides preferably have an L-glutamine content of 20-40 wt% and an average molecular weight of 500-20,000, and most preferably are peptides produced from wheat gluten as a raw material.

In the description which follows, peptides having high glutamine content according to the invention will be referred to as "glutamine peptides". Throughout the present specification, the terms "peptides" and "glutamine peptides" refer to peptides composed of L-amino acids found in natural proteins. Thus, the terms "L-amino acids" and "amino acids" refer to L-amino acids found in natural proteins.

The present invention further relates to foods containing peptides having prophylactic and/or therapeutic effects on liver diseases, hyperlipemia and/or diabetes. Addition of glutamine peptides of the invention to foods and beverages allows the glutamine peptides to be more easily ingested during daily activities.

In the invention, "liver disease" is fatty liver or inflammatory hepatitis such as viral hepatitis, alcoholic hepatitis or hepatic cirrhosis, and includes symptoms which may not be clearly identified but are associated with abnormal value of liver function indices such as GOT, GTP and $\gamma$-GTP.

GOT (Glutamic Oxaloacetic Transaminase) is an enzyme found abundantly in cells of tissues and organs such as the heart, liver, skeletal muscle, kidneys, etc., which decomposes protein into amino acids. Since formation and disintegration occur simultaneously in cells of the human body even in a normal state, a certain level of GOT is always present in blood of healthy persons. The GOT content (normal value) in blood of healthy adults is generally in range of 5-40 IU/liter, but abnormality in the tissues or organs such as the heart, liver, skeletal muscle or kidneys results in massive release of GOT into the blood. The GOT value is therefore one of the important indices for diagnosis of pathological conditions in the organs or body tissues.

GPT (Glutamic Pyruvic Transaminase), like GOT, which is an enzyme associated with amino acid formation, is specifically abundant in liver cells, while kidney is about 1/3 that in liver and very low in other organs. A normal GPT blood level in adults is generally in the range of 5-42 IU/liter, but GPT level (GPT value) in blood increases in a highly sensitive manner in response to hepatopathy including liver cell degeneration or necrosis, and it is an important index for diagnosis of liver disease.

γ-GTP (γ-Glutamyl Transpeptidase), like GOT and GPT, which is a protein-degrading enzyme, is most abundant in the kidneys while also being found in the pancreas, liver, spleen and small intestine. In liver, γ-GTP is abundantly present at regions associated with secretion and absorption of enzymes, including the bile capillaries, biliary epithelium and intestinal epithelium cells. The normal blood level for γ-GTP in adults is generally no greater than 50 IU/liter (0-50 IU/liter). γ-GTP is released into the blood in greater amounts upon destruction of liver cells by alcohol or drugs with liver toxicity, or when the bile duct interior is blocked by calculus, tumor or the like. γ-GTP is particularly sensitive to alcohol, and because diseases of liver or biliary tract also produce abnormalities in this enzyme earlier than in other enzymes, γ-GPT is widely used as an index of liver disease together with GOT and GPT.

In the invention, "hyperlipemia" means a condition where blood cholesterol, triglycerides or both exceed specified standard values, and the condition of hyperlipemia includes hypercholesterolemia and hypertriglyceridemia. Thus, prophylactic or therapeutic agents for hyperlipemia according to the invention are administered or taken for treatment or prevention of conditions in which cholesterol or triglycerides have exceeded the specified standard values. The indices for hyperlipemia include not only total cholesterol (total of free cholesterol and esterified cholesterol) and triglycerides (triglyceride value), but also LDL cholesterol (low-density lipoprotein cholesterol value) .

In the invention, "diabetes" means a condition in which blood sugar level has exceeded a specified standard value. Thus, prophylactic or therapeutic agents for diabetes according to the invention are administered or taken for treatment or prevention of conditions in which the blood glucose level has exceeded the standard value. The indices for diabetes include not only blood glucose level (for example, fasting blood glucose), but also glycohemoglobin A1c (HbAlc) and 1,5-anhydro-D-glucitol (1,5-AG) .

HbAlc which is the glucose-bound form of hemoglobin A (HbA), includes HbAlc unstable form and HbAlc stable form. Stable HbAlc is stably bound with glucose and indicates blood glucose level changes from 4 months previous, but it is usually used to determine average blood glucose levels from 1-2 months previous. The normal value of HbAlc is in range of 4.3-5.8%, with some sort of treatment being required if the value exceeds 6.0%.

1,5-Anhydro-D-glucitol (1,5-AG) is a monosaccharide which is partially reduced glucose. The normal value range for 1,5-AG is at least 14 µg/mL, but it decreases drastically upon excretion of urinary sugar due to hyperglycemia and is gradually restored as blood glucose condition improves. It therefore indicates changes in blood glucose during a short period of from 5-7 days previous, and it can be evaluated at any time since it is not affected by meals, etc.

Methods for measuring the indices of blood glucose, HbAlc and 1,5-AG are widely known in the field of diabetes treatment, and their standards have been established. Measuring devices and test kits are commercially available and may be used for measurement.

The present invention will be explained in detail.

The glutamine peptides used for the invention are peptides having an L-glutamine content of at least 15 wt% and an average molecular weight of 200-100,000, but not particularly limited thereto, and they may be partial hydrolysates of natural or recombinant protein, chemically synthesized or genetically engineered peptides, or combinations thereof.

If the L-glutamine content of the glutamine peptides is less than 15 wt%, an adequate prophylactic and/or therapeutic effect for the disease may not be achieved. There is no particular upper limit on the L-glutamine content, but a content exceeding 60 wt% may complicate preparation from natural protein, and therefore the content is preferably no greater than 60 wt% from the standpoint of ready availability and preparation as well as economy, while a range of 20-40 wt% is more preferred.

The L-glutamine content of the glutamine peptides may be evaluated by a calculating method based on the amide nitrogen-containing L-amino acid content, where the amide nitrogen content is measured by the amide nitrogen substitution method or, when the glutamine peptides are a synthesized product, by the proportion of L-glutamine used for the synthesis.

If the average molecular weight of the glutamine peptides is less than 200, an undesirable bitter taste will be exhibited. On the other hand, if the average molecular weight of the glutamine peptides exceeds 100,000, a viscous mass will be produced upon addition of water, thereby impairing the manageability. The average molecular weight of the peptides is within the range of 200-100,000, preferably 500-20,000 and more preferably 1000-10,000, but not particularly limited thereto.

The "average molecular weight" of peptides as referred to the specification is the average molecular weight as measured by gel filtration, the details of which are illustrated in Examples.

The glutamine peptides used for the invention are not particularly limited in regard to species and contents of L-amino acids other than L-glutamine, provided that the L-glutamine content is at least 15 wt%.

Because glutamine peptides are composed of L-amino acids found in natural proteins, they are superior in terms of safety in humans and animals, but more preferably in terms of safety, availability and ease and economy of preparation, the peptides of the invention are glutamine peptides prepared by hydrolysis of proteins found in foods, and most preferably glutamine peptides prepared by hydrolysis of wheat gluten.

The peptides preferably used in the invention may be produced by hydrolysis of wheat gluten which comprises 25-50 wt% L-glutamine, using proteases, acids, alkalis or the like under conditions allowing production of hydrolysates with an average molecular weight in the range of 200-100,000.

Since wheat gluten normally comprises 25-50 wt% L-glutamine, hydrolysis of wheat gluten under conditions producing an average molecular weight in the range of 200-100,000, with fractionation and the like if necessary, can yield glutamine peptides having an L-glutamine content in the range of 15-60 wt% in a relatively easy manner.

Wheat gluten is insoluble and poorly dispersible in water, but glutamine peptides prepared by hydrolysis of gluten are highly dispersible in water because of their low molecular weight.

Wheat gluten-derived glutamine peptides can be produced, for example, by a method of hydrolyzing gluten using proteases or using proteases and amylases (see Japanese Patent No. 2019439 and Japanese Patent No. 2985193). Examples of proteases which may be used include pepsin, trypsin, chymotrypsin, orange polypore-derived acid proteases, Aspergillus-derived acid proteases, papain, bromelain and the like. Combined use of amylases during the hydrolysis may decompose and remove impurities such as starches and fiber contained in the gluten, to afford high-purity glutamine peptides at a high yield (see Japanese Patent No. 2985193).

Glutamine peptides of the invention, for example, gluten-derived glutamine peptides, are already commercially available (for example, "Glutamine Peptide GP-1" by Nisshin Pharma Inc., and "Glutamine Peptide GPA" by DMV Intl.). These glutamine peptides may be used directly, or maybe subjected to hydrolysis, fractionation or the like depending on the case.

Prophylactic or therapeutic agents and foods for liver diseases, hyperlipemia and/or diabetes according to the invention will generally be administered in the range of 1-40 g per day for adults, in terms of glutamine peptide mass. The ordinary daily dosage is 6-10 g. However, since the peptides used for the invention are naturally-derived and highly safe, their dosages may be even higher. The dosage is preferably adjusted as suitable for the desired effect. The daily dosage may be administered or taken at once, but is preferably divided into multiple doses.

The prophylactic or therapeutic agents according to the invention may be prepared as oral formulations such as tablets, granules, powders, capsules, syrups, dry syrups, liquids or the like according to established methods. If necessary, other publicly known additives such as excipients, binders, disintegrators, lubricants, aromatics, colorants, surfactants, coating agents and the like may also be used.

Examples of excipients include glucose, saccharose, lactose, mannitol, xylitol, sorbitol, crystalline cellulose, microcrystalline cellulose, carboxymethylcellulose sodium, starches, carboxymethyl starch sodium, dextrin, cyclodextrin, carboxyvinyl polymer, calcium hydrogen phosphate, light silicic anhydride, titanium oxide and magnesium aluminate metasilicate.

Examples of binders include tragacanth gum, gum arabic, corn starch, gelatin, alpha starch, sodium alginate, methyl cellulose, ethyl cellulose, hydroxypropyl cellulose, hydroxypropylmethyl cellulose, polyvinylpyrrolidone and polyvinyl alcohol. Examples of disintegrators include starch, surfactants, and sodium hydrogen carbonate which react with gastric acid to produce gas. Examples of lubricants include stearic acid, magnesium stearate, calcium stearate, talc, hydrogenated oil, sucrose fatty acid ester, dimethylpolysiloxane, microcrystalline wax and beeswax.

Examples of aromatics include acidifiers such as citric acid, sweeteners such as saccharin, and aromatic substances such as peppermint. As colorants, pigments approved for use in foods may be used.

Examples of surfactants include polyoxyethylene hydrogenated castor oil, glycerin monostearate, sorbitan monostearate, sorbitan monopalmitate, sorbitan monolaurate, polysorbates, sodium lauryl sulfate and sucrose fatty acid esters. Examples of coating agents include methyl cellulose, ethyl cellulose and hydroxypropyl cellulose.

Addition of glutamine peptides of the invention to foods will further facilitate their ingestion. Examples of such foods include carbohydrate-based foods such as bread, noodles such as Chinese udon noodles, cookies, crackers and the like, teas, soups, seasonings, spreads such as butter and jams, liquid foods, juices, jelly beverages and the like. Health foods and functional foods are preferably in forms which allow management of daily dosages. Foods according to the invention have effects of preventing or treating liver diseases, hyperlipemia and/or diabetes.

Best Mode for Carrying Out the Invention

The present invention will be illustrated through the following examples, but not limited thereto.

The average molecular weights and the glutamine contents of the peptides (glutamine peptides) were measured by the following methods.

Peptide average molecular weight:

An aqueous solution of the peptide was filtered with a 0.45 μm membrane filter and the molecular weight was measured by gel filtration. The column used was "BIOSIL SEC125-5" by Nihon Biorad Laboratories. The measuring conditions were a measuring wavelength of 280 nm, a 0.2 mM phosphate buffer solution as eluent (pH 6.0, 0.1% SDS) and a flow rate of 0.2 ml/min. The molecular weight standards were ovalbumin (molecular weight: 44 kDa), myoglobulin (molecular weight: 17 kDa) and vitamin B12 (molecular weight: 1.35 kDa).

Peptide glutamine content:

Glutamine and asparagine are both amide nitrogen-containing amino acids, but ≥95 wt% of the amide nitrogen-containing amino acids in wheat protein are glutamine. Thus, no significant error will result if the amide nitrogen-containing amino acid content of wheat gluten is considered to be the glutamine content. The amide nitrogen-containing amino acid content of the gluten was therefore directly recorded as the glutamine content.

The amide nitrogen-containing amino acid content was determined according to the Wilcox assay for amides in chemical substances [Meth. Enzymol., 11, 36-65(1967)]. Specifically, the glutamine peptides were placed in a Conway flask, 1 N hydrochloric acid was added to liberate the amide nitrogen in the glutamine peptides as ammonia, the generated ammonia was quantitated according to hygienic guidelines ["Hygienic Test Methods and Commentary" ed. by Pharmaceutical Society of Japan, p.274-276, Kanehara & Co., Ltd. (1990)], upon which the amide nitrogen content in the peptides was determined based on the assayed ammonia content, and this was used to calculate the amide nitrogen-containing amino acid content as the glutamine content.

Production Example 1 Production of glutamine peptides

(1) After charging 9700 kg of ion-exchanged water, 38 kg of citric anhydride and 1500 kg of wheat gluten (active gluten, Weston Foods Limited) in a reaction tank, the mixture was heated to 45°C, and then 2.2 kg of protease ("Protease M Amano", Amano Pharmaceutical Co., Ltd.) and 1.1 kg of amylase ("Liquefied Enzyme T", HBI Co., Ltd.) were added and hydrolysis reaction was conducted for 5 hours at 45°C, after which a 25% aqueous sodium hydroxide solution was used to adjust pH of the solution to 4.4-4.5 before holding it for 5 hours for enzyme treatment.
(2) The solution was then held at 80°C for 20 minutes for inactivation of the protease, and then after cooling to 65°C, 0.5 kg of amylase ("Liquefied Enzyme T", HBI Co., Ltd.) was added for hydrolysis of starches and fibrous matter in the gluten, and the solution was then held at 90°C for 20 minutes for inactivation of the amylase.
(3) Next, the solution was cooled to below 10°C and subsequently reheated to 55°C, and then 100 kg of active carbon ("TAKECORE", Takeda Chemical Industries, Ltd.) was added and the mixture was stirred at 55°C for 30 minutes.
(4) The liquid temperature was adjusted to 45°C, a filtering aid ("RADIOLITE", Showa Chemical Industry Co., Ltd.) was added, and filtration was performed using a pressure filtration apparatus to afford filtrate of 7000 liters (7 m$^3$).
(5) The filtrate afforded in (4) was concentrated under reduced pressure to a Brix value of 20-40, and then heated using a plate heater at 110°C for 20 seconds for sterilization and cooled to 55°C.
(6) The solution afforded in (5) was spray dried using a spray drier with a blowing temperature of 160°C and an exhaust temperature of 80°C, to give approximately 1000 kg of glutamine peptide powder as a wheat gluten hydrolysate.
(7) The glutamine peptide powder obtained in (6) was classified using a 60 mesh sieve (0.246 mm holes), and the fine powder passing through the sieve (glutamine peptide fine powder) was collected.
(8) The average molecular weight and glutamine content of the peptide (fine powder) collected in (7) were measured. The results were an average molecular weight of 8000 and a glutamine content of 32 wt%.

Production Examples 2-4 Production of glutamine peptides

Glutamine peptides having the properties shown in Table 1 were prepared as the same manner of Production Example 1.

Table 1

|  | Average molecular weight | Glutamine content |
| --- | --- | --- |
| Production Example 2 | 300 | 25 wt% |
| Production Example 3 | 10,000 | 32 wt% |
| Production Example 4 | 16,000 | 34 wt% |

Example 1 Production of tablets

After mixing 83.3 g of the glutamine peptides prepared in Production Example 1, 10 g of crystalline cellulose (Asahi Kasei Co., Ltd.) and 5 g of polyvinylpyrrolidone (BASF), 30 ml of ethanol was added to the mixture and granules were produced by an ordinary wet method. The granules were dried, and then 1.2 g of magnesium stearate was added to prepare granular powder for tableting, and a tableting machine was used to produce 100 tablets at 1 g each (glutamine peptide content per tablet: 0.838 g).

Example 2 Production of syrup

After adding and dissolving 750 g of saccharose and 100 g of glutamine peptides prepared in Production Example 1 in 400 g of purified water while boiling and stirring, the solution was percolated while hot, and purified water was added thereto to a total volume of 1000 ml to produce a syrup (glutamine peptide content per 100 ml of syrup: 10 g).

Example 3 Production of granular preparation

After mixing 76 g of glutamine peptide GP-1 (average molecular weight: 7000, L-glutamine content: 32 wt%, Nisshin Pharma Inc.), 13.3 g of lactose (DMV Co.), 6.7 g of crystalline cellulose (Asahi Kasei Co., Ltd.) and 4 g of polyvinylpyrrolidone (BASF), 30 ml of ethanol was added to the mixture, and then granules were produced by an ordinary wet method and dried and further sieved to afford a granular preparation (glutamine peptide content per 10 g of granular preparation: 7.6 g).

Example 4 Production of liquid food

After adding and dissolving 40 g of casein sodium (DMV Co.), 160 g of maltodextrin (Sanwa Cornstarch Co.) and 25 g of glutamine peptide GP-1 (Nisshin Pharma Inc.) in 750 g of purified water at approximately 65°C, a mixed solution containing 5 g of vitamin mix and trace minerals was added. The mixture was loaded into a homomixer (Tokushu Kika Kogyo Co., Ltd.) and crudely emulsified for 15 minutes at about 8000 rpm. The obtained emulsion was cooled to about 20°C, an aromatic was added, and the meniscus was adjusted to the measuring level. After filling a 230 g portion of the solution into a pouch, the pouch was sealed while performing nitrogen replacement, and sterilization was carried out at 121°C for 15 minutes to obtain a concentrated liquid food. The glutamine peptide content per 230 g of liquid food was approximately 5 g.

Example 5 Preparation of bread

A 150 g portion of wheat flour (strong flour) was mixed with 2 g of dry yeast. Separately, 20 g of glutamine peptide GP-1 (Nisshin Pharma Inc.), 20 g of sugar, 3 g of salt and 6 g of skim milk powder were dissolved in 70 g of warm water, and after adding one egg, the mixture was thoroughly stirred. This was added to the wheat flour, and after thorough kneading by hand, approximately 40 g of butter was added and kneading was continued to prepare 20 bread roll doughs. This was followed by fermentation, the surface was coated with a beaten egg, and the dough was baked for about 15 minutes in an oven at 180°C to produce bread rolls. The bread rolls contained about 1 g of glutamine peptides per roll.

Example 6 Preparation of meat sauce for pasta

A single portion (150 g) of pasta meat sauce was placed in a pot while adding 5 g of glutamine peptide GP-1 (Nisshin Pharma Inc.), and then the mixture was heated to prepare meat sauce for pasta. The sauce was filled into a pouch, and the pouch was then sealed while performing nitrogen replacement, and sterilization was carried out at 121°C for 15 minutes to obtain glutamine peptide-containing pasta meat sauce.

Example 7 Preparation of Chinese udon noodles

A dispersion of 15 g of glutamine peptide GP-1 (Nisshin Pharma Inc.) and 15 g of salt in 150 g of water was thoroughly kneaded with 300 g of wheat flour (all purpose flour) and the kneaded mixture was allowed to stand. The dough was then pressed and cut to widths of approximately 5 mm to produce udon noodles. Upon boiling and simmering for 10 minutes in hot water, the outer appearance, flavor and texture were satisfactory. The udon noodles contained approximately 5 g of glutamine peptides per serving.

Treatment of liver disease, hyperlipemia and/or diabetes using glutamine peptides of the invention will now be explained through the following test examples.

[Tests for liver disease]

Pharmacological tests using glutamine peptides of the invention for liver disease will now be described.
The methods of measuring the GOT, GPT and γ-GTP values as indices of therapeutic effects for liver disease were as follows.
GOT: Measured according to a UV method ["Rinsho Kagaku" 18, p.231-249 (1989)], as the standardized method of the Japan Society of Clinical Chemistry (JSCC).
GPT: Measured by a UV method ["Rinsho Kagaku" 18, p.250-262 (1989)], as the standardized method of the (JSCC).
γ-GPT: Measured by a colorimetric method ["Rinsho Kagaku" 24, p.106-121 (1995)], as the standardized method of the (JSCC).

[Test Example 1 for fatty liver]

Subject A: male (Age 58)
Drug: Granular preparation of Example 3
Dosage: Approximately 9.9 g/day glutamine peptide

Method: The fatty liver subject, complaining of general fatigue and loss of appetite, was administered glutamine peptides three times a day after meals, and the GOT, GPT and γ-GTP were measured before and 1 and 3 weeks after the start of administration. The results are shown in Table 2.

Table 2

|  | Before administration | 1 week after administration | 3 weeks after administration |
|---|---|---|---|
| GOT (IU/l) (normal: 5-40 IU/l) | 72 | 35 | 16 |
| GPT (IU/l) (normal: 5-42 IU/l) | 124 | 85 | 25 |
| γ-GTP (IU/l) (normal: 0-50 IU/l) | 70 | 47 | 31 |

[Test Example 2 for fatty liver]

Subject B: male (Age 60)
Drug: Syrup of Example 2
Dosage: Approximately 6 g/day glutamine peptide

Method: The fatty liver subject, complaining of fatigue and loss of appetite, was administered glutamine peptides three times a day after meals, and the GOT, GPT and γ-GTP were measured before and 3 weeks after the administration. The results are shown in Table 3.

Table 3

|  | Before administration | 3 weeks after administration |
|---|---|---|
| GOT (IU/l) (normal: 5-40 IU/l) | 56 | 33 |
| GPT (IU/l) (normal: 5-42 IU/l) | 109 | 56 |
| γ-GTP (IU/l) (normal: 0-50 IU/l) | 140 | 60 |

[Test Example 1 for chronic hepatitis C]

Subject C: male (Age 49)
Drug: Syrup of Example 2
Dosage: Approximately 8 g/day glutamine peptide

Method: The chronic hepatitis C subject was administered glutamine peptides two times a day (morning/ evening), and the GOT, GPT and γ-GTP were measured before and 3 weeks after the administration. The results are shown in Table 4.

Table 4

|  | Before administration | 3 weeks after administration |
|---|---|---|
| GOT (IU/l) (normal: 5-40 IU/l) | 73 | 47 |
| GPT (IU/l) (normal: 5-42 IU/l) | 58 | 26 |
| γ-GTP (IU/l) (normal: 0-50 IU/l) | 68 | 57 |

[Test Example 2 for chronic hepatitis C]

Subject D: male (Age 51)
Drug: Granular preparation of Example 3
Dosage: Approximately 8 g/day glutamine peptide

Method: The chronic hepatitis C subject was administered glutamine peptides two times a day (morning/ evening), and the GOT, GPT and γ-GTP were measured before and 4 weeks after the administration. The results are shown in Table 5.

Table 5

|  | Before administration | 4 weeks after administration |
|---|---|---|
| GOT (IU/l) (normal: 5-40 IU/l) | 81 | 39 |
| GPT (IU/l) (normal: 5-42 IU/l) | 176 | 62 |
| γ-GTP (IU/l) (normal: 0-50 IU/l) | 51 | 37 |

Both of the subjects in these tests exhibited reduced GOT, GPT and γ-GPT values as liver function markers, thus indicating improved liver function. The results suggested that the glutamine peptides of the invention have excellent pharmacological effects for prevention or treatment of liver diseases.

[Tests for hyperlipemia]

Pharmacological tests using glutamine peptides of the invention for hyperlipemia will now be described.
Subjects with hyperlipemia were orally administered specific amounts of glutamine peptides in correspondence to their symptoms, and after a prescribed period of time the total cholesterol, triglycerides, LDL cholesterol and arteriosclerosis index values were measured to evaluate the improving effect on hyperlipemia. The blood total cholesterol, LDL cholesterol, triglyceride and arteriosclerosis index values were measured by the following methods.
Total cholesterol: Measured according to an enzyme method [Uji et al., Nihon Rinsho, Vol.53, Supplementary Edition, p.615 (1995)], by the clinical laboratory trustee SRL Co., Ltd. (Tokyo).
LDL cholesterol: Measured according to an enzymatic method [Kanno et al., Igaku to Yakugaku, Vol.3, No.3, p.635 (1997)], by the clinical laboratory trustee SRL Co., Ltd. (Tokyo) .
Triglycerides: Measured according to an enzyme method (free glycerol elimination method) [Shibuya et al., Nihon Rinsho, Vol.53, Supplementary Edition, p.606 (1995)], by the clinical laboratory trustee SRL Co., Ltd. (Tokyo). Arteriosclerosis index: Measurement was conducted according to an HDL cholesterol selective inhibition method (direct method) [Nakai et al., Nihon Rinsho, Vol.53, Supplementary Edition, p.617 (1995)], by the clinical laboratory trustee SRL Co., Ltd. (Tokyo), and the arteriosclerosis index was measured by the following formula:

Arteriosclerosis index = (Total cholesterol - HDL cholesterol)/(HDL cholesterol)

[Test Example 1 for hyperlipemia]

Subject E: male (Age 58)
Drug: Granular preparation of Example 3
Dosage: 10 g/day glutamine peptide

Method: The subject was administered glutamine peptides three times a day after meals, and the total cholesterol and triglycerides were measured before and 1 week, 3 weeks and 3 months after the administration. The results are shown in Table 6.

Table 6

| | Before administration | 1 week after administration | 3 weeks after administration | 3 months after administration |
|---|---|---|---|---|
| Total cholesterol (mg/dl) (Normal: 130-220 mg/dl) | 178 | 177 | 180 | 152 |
| Triglycerides (mg/dl) (Normal: 30-149 mg/dl) | 218 | 181 | 159 | 149 |

[Test Example 2 for hyperlipemia]

Subject F: male (Age 60)
Drug: Granular preparation of Example 3
Dosage: 6 g/day glutamine peptide

Method: The subject was administered glutamine peptides three times a day after meals, and the total cholesterol, LDL cholesterol, triglycerides and arteriosclerosis index were measured before and 3 weeks and 3 months after the administration. The results are shown in Table 7.

Table 7

| | Before administration | 3 weeks after administration | 3 months after administration |
|---|---|---|---|
| Total cholesterol (mg/dl) (Normal: 130-220 mg/dl) | 256 | 243 | 224 |
| LDL cholesterol (mg/dl) (Normal: ≤140 mg/dl | 157 | 129 | 124 |
| Triglycerides (mg/dl) (Normal: 30-149 mg/dl) | 232 | 307 | 223 |
| Arteriosclerosis index (Normal: ≤3) | 3.8 | 3.6 | 3.1 |

Both of the subjects in these tests exhibited reduced total cholesterol, triglyceride, LDL cholesterol and arteriosclerosis index values as hyperlipemia markers, thus indicating improvement of hyperlipemia. The results suggested that the glutamine peptides of the invention have excellent pharmacological effects for prevention or treatment of hyperlipemia.

[Tests for diabetes]

Pharmacological tests using glutamine peptides of the invention for diabetes will now be described.

Subjects with diabetes were orally administered specific amounts of glutamine peptides in correspondence to their symptoms, and after a prescribed period of time the fasting blood glucose, HbAlc and 1,5-AG levels were measured to evaluate the improving effect on diabetes. The blood glucose, HbA1c and 1,5-AG levels were measured by the following methods.

Blood glucose: Common blood glucose measuring methods include enzymatic electrode methods such as the glucose oxidase (GOD) method and glucose dehydrogenase (GDH) method, and enzyme colorimetric methods such as the hexokinase (HX) method and the glucose oxidase/peroxidase (GOD/POD) method, but for these examples the measurement was performed by the GDH method [Maekawa et al., Nihon Rinsho, Vol.53, Supplementary Edition, p.527 (1995)], by the clinical laboratory trustee SRL Co., Ltd. (Tokyo).

HbA1c: Standard methods for HbAlc include high performance liquid chromatography (HPLC) methods, latex agglutination (LA) methods and affinity methods, but for these examples the measurement was performed an LA method [Oimomi et al., Nihon Rinsho, Vol.48, Supplementary Edition, p.315 (1990)], by the clinical laboratory trustee SRL Co., Ltd. (Tokyo). 1,5-AG: For these examples, the measurement was performed by an enzyme method [Kawai et al., Nihon Rinsho, Vol.47, Supplementary Edition, p.439 (1989)], by the clinical laboratory trustee SRL Co., Ltd. (Tokyo).

[Test example for diabetes]

Subject G: male (Age 67)
Drug: Granular preparation of Example 3
Dosage: 10 g/day glutamine peptide

Method: The subject was administered glutamine peptides three times a day after meals, and the fasting blood glucose, HbA1c and 1,5-AG levels were measured before and 1 and 3 months after the administration. The results are shown in Table 8.

Table 8

|  | Before administration | 1 month after administration | 3 months after administration |
|---|---|---|---|
| Fasting blood glucose (mg/dl) (Normal: 70-110 mg/dl) | 157 | 138 | 127 |
| HbA$_{1c}$ % (Normal: 4.3-5.8%) | 7.4 | 6.6 | 6.4 |
| 1,5-AG (mg/dl) (Normal: ≥14 µg/ml) | 5.3 | 7.2 | 10.9 |

The subject exhibited notably improved fasting blood glucose, HbAlc and 1,5-AG values as diabetes markers. The results suggested that the glutamine peptides of the invention have excellent pharmacological effects for prevention or treatment of diabetes.

[Test Example 1 for coincident fatty liver and hyperlipemia]

Subject H: male (Age 68)
Drug: Granular preparation of Example 3
Dosage: Approximately 8 g/day glutamine peptide

Method: The subject, having coincident fatty liver and hyperlipemia and complaining of fatigue and loss of appetite, was administered glutamine peptides two times a day (morning/evening), and the GOT, GPT, $\gamma$-GTP, total cholesterol, LDL cholesterol, triglycerides and arteriosclerosis index were measured before and 4 weeks and 3 months after the administration. The results are shown in Table 9.

Table 9

|  | Before administration | 4 weeks after administration | 3 months after administration |
|---|---|---|---|
| GOT (IU/l) (normal: 5-40 IU/l) | 70 | 37 | 31 |
| GPT (IU/l) (normal: 5-42 IU/l) | 124 | 67 | 38 |
| $\gamma$-GTP (IU/l) (normal: 0-50 IU/l) | 103 | 58 | 49 |
| Total cholesterol (mg/dl) (Normal: 130-220 mg/dl) | 293 | 293 | 211 |
| LDL cholesterol (mg/dl) (Normal: ≤140 mg/dl | 193 | 198 | 134 |
| Triglycerides (mg/dl) (Normal: 30-149 mg/dl) | 272 | 244 | 187 |
| Arteriosclerosis index (Normal: ≤3) | 5.4 | 5.4 | 4.3 |

[Test Example 2 for coincident fatty liver and hyperlipemia]

    Subject I: male (Age 50)
Drug: Granular preparation of Example 3
Dosage: Approximately 8 g/day glutamine peptide

Method: The subject, exhibiting signs of fatty liver and having hyperlipemia, was administered glutamine peptides two times a day (morning/evening), and the GOT, GPT, $\gamma$-GTP, total cholesterol, LDL cholesterol, triglycerides and arteriosclerosis index were measured before and 4 weeks and 3 months after the administration. The results are shown in Table 10.

Table 10

|  | Before administra - tion | 4 weeks after administration | 3 months after administration |
|---|---|---|---|
| GOT (IU/l) (normal: 5-40 IU/l) | 23 | 24 | 20 |
| GPT (IU/l) (normal: 5-42 IU/l) | 43 | 29 | 22 |
| $\gamma$-GTP (IU/l) (normal: 0-50 IU/l) | 61 | 27 | 28 |
| Total cholesterol (mg/dl) (Normal: 130-220 mg/dl) | 215 | 157 | 159 |
| LDL cholesterol (mg/dl) (Normal: ≤140 mg/dl | 105 | 68 | 87 |
| Triglycerides (mg/dl) (Normal: 30-149 mg/dl) | 321 | 221 | 124 |

Table 10   (continued)

|  | Before administra - tion | 4 weeks after administration | 3 months after administration |
|---|---|---|---|
| Arteriosclerosis index (Normal: ≤3) | 3.7 | 2.5 | 2.4 |

Both of the subjects in these tests exhibited reduced GOT, GPT and γ-GTP values as liver function markers and reduced total cholesterol, triglyceride, LDL cholesterol and arteriosclerosis index values as hyperlipemia markers, thus indicating improvement in liver function and hyperlipemia. The results suggested that the glutamine peptides of the invention have excellent effects even for prevention or treatment of coincident fatty liver and hyperlipemia.

[Test example for coincident hyperlipemia and diabetes]

Subject J: female (Age 74)
Drug: Granular preparation of Example 3
Dosage: 6 g/day glutamine peptide

Method: The subject was administered glutamine peptides three times a day after meals, and the total cholesterol, LDL cholesterol, triglycerides, fasting blood glucose, HbAlc and 1,5-AG levels were measured before and 2 weeks, 4 weeks and 3 months after the administration. The results are shown in Table 11.

Table 11

|  | Before administration | 2 weeks after administration | 4 weeks after administration | 3 months after administration |
|---|---|---|---|---|
| Total cholesterol (mg/dl) (Normal: 130-220 mg/dl) | 226 | 180 | 194 | 184 |
| LDL cholesterol (mg/dl) (Normal: ≤140 mg/dl | 128 | 101 | 111 | 104 |
| Triglycerides (mg/dl) (Normal: 30-149 mg/dl) | 122 | 87 | 102 | 83 |
| Fasting blood glucose (mg/dl) (Normal: 70-110 mg/dl) | 146 | 128 | 118 | 116 |
| HbA$_{1c}$ % (Normal: 4.3-5.8%) | 8.1 | 7.1 | 6.4 | 6.1 |
| 1,5-AG (mg/dl) (Normal: ≥14 μg/ml) | 5.4 | 12.8 | 16.5 | 14.2 |

The subject in this test exhibited notably reduced total cholesterol, triglyceride and LDL cholesterol values as hyperlipemia markers and notably improved fasting blood glucose, HbA1c and 1,5-AG values as diabetes markers. The results suggested that the glutamine peptides of the invention have excellent effects even for prevention or treatment of coincident hyperlipemia and diabetes.

Industrial Applicability

According to the present invention there are provided prophylactic and/or therapeutic agents for liver diseases, hyperlipemia and/or diabetes which comprises peptides composed of L-amino acids found in natural proteins, having an L-glutamine content of 15-60 wt% and an average molecular weight of 200-100,000, as well as foods containing the peptides.

Drugs or foods of the invention may be administered or ingested to achieve notable improvement in liver disease, hyperlipemia and/or diabetes within a short period of time. The glutamine peptides used for the invention are also highly safe and suitable for prolonged administration. Drugs and foods of the invention are therefore extremely useful for prevention and/or treatment of liver disease, hyperlipemia and/or diabetes.

**Claims**

1.  A prophylactic and/or therapeutic agent for a liver disease, hyperlipemia and/or diabetes which comprises peptides composed of L-amino acids found in natural proteins, having an L-glutamine content of 15-60 wt% and an average molecular weight of 200-100,000.

2.  The prophylactic and/or therapeutic agent according to claim 1, wherein the peptide has the L-glutamine content of 20-40 wt% and the average molecular weight of 500-20,000.

3.  The prophylactic and/or therapeutic agent according to claim 1 or 2, wherein the peptide is produced from wheat gluten as the raw material.

4.  The prophylactic and/or therapeutic agent according to any one of claims 1 to 3, wherein the liver disease is fatty liver or chronic hepatitis C.

5.  The prophylactic and/or therapeutic agent according to any one of claims 1 to 3, wherein the hyperlipemia is hypercholesterolemia or hypertriglyceridemia

6.  A food for prevention or treatment of a liver disease, hyperlipemia and/or diabetes which contains a peptide according to any one of claims 1 to 3.

**EP 1 481 684 A1**

<table>
<tr><td colspan="2" style="text-align:center"><strong>INTERNATIONAL SEARCH REPORT</strong></td><td>International application No.<br>PCT/JP03/02366</td></tr>
</table>

| A. CLASSIFICATION OF SUBJECT MATTER |
| --- |
| Int.Cl$^7$  A61K38/16, A61P1/16, 3/06, 3/10 |

According to International Patent Classification (IPC) or to both national classification and IPC

| B. FIELDS SEARCHED |
| --- |
| Minimum documentation searched (classification system followed by classification symbols)<br>Int.Cl$^7$  A61K38/16, A61P1/16, 3/06, 3/10 |

Documentation searched other than minimum documentation to the extent that such documents are included in the fields searched

| | | | |
|---|---|---|---|
| Jitsuyo Shinan Koho | 1926–1992 | Toroku Jitsuyo Shinan Koho | 1994–1996 |
| Kokai Jitsuyo Shinan Koho | 1971–1992 | Jitsuyo Shinan Toroku Koho | 1996–2003 |

Electronic data base consulted during the international search (name of data base and, where practicable, search terms used)
CA(STN), MEDLINE(STN), BIOSIS(STN), EMBASE(STN)

| C. DOCUMENTS CONSIDERED TO BE RELEVANT |
| --- |

| Category* | Citation of document, with indication, where appropriate, of the relevant passages | Relevant to claim No. |
|---|---|---|
| X<br>Y | JP 2002-20312 A  (Meiji Seika Kaisha, Ltd.),<br>23 January, 2002 (23.01.02),<br>Full text<br>(Family: none) | 1,2,4-6<br>3 |
| Y | JP 7-255398 A  (Snow Brand Milk Products Co., Ltd.),<br>09 October, 1995 (09.10.95),<br>Full text<br>(Family: none) | 3 |
| Y | JP 6-245790 A  (Nisshin Flour Milling Co., Ltd.),<br>06 September, 1994 (06.09.94),<br>Full text<br>(Family: none) | 3 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>27 May, 2003 (27.05.03) | Date of mailing of the international search report<br>10 June, 2003 (10.06.03) |
|---|---|
| Name and mailing address of the ISA/<br>Japanese Patent Office | Authorized officer |
| Facsimile No. | Telephone No. |

Form PCT/ISA/210 (second sheet) (July 1998)

15

**EP 1 481 684 A1**

<table>
<tr><td colspan="2" align="center">INTERNATIONAL SEARCH REPORT</td><td>International application No.<br>PCT/JP03/02366</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| Y | JP 5-236909 A  (Snow Brand Milk Products Co., Ltd.), 17 September, 1993 (17.09.93), Full text (Family: none) | 3 |

Form PCT/ISA/210 (continuation of second sheet) (July 1998)